# EUROPEAN PATENT SPECIFICATION

(11) **EP 1 926 527 B1**
(45) Date of publication and mention of the grant of the patent: **13.11.2013**
(21) Application number: 06824949.9
(22) Date of filing: 13.09.2006
(51) Int. Cl.: A61N 1/36

(54) **TRANS-RETINAL FLEXIBLE CIRCUIT ELECTRODE ARRAY**
TRANSRETINALE FLEXIBLE KREISLAUF-ELEKTRODEN-ANORDNUNG
RESEAU D'ELECTRODES A CIRCUIT SOUPLE TRANSRETINIEN

(30) Priority: 19.09.2005 US 718779 P
(43) Date of publication of application: 04.06.2008
(60) Divisional application: 11172502.4
(73) Proprietor: Second Sight Medical Products, Inc., Sylmar, CA 91342 (US); DOHENY EYE INSTITUTE, Los Angeles, CA 90033 (US)
(72) Inventor: GREENBERG, Robert, J., Los Angeles, CA 90068 (US); HUMAYUN, Mark, S., Glendale, CA 91206 (US)
(74) Representative: Carter, Stephen John
(86) International application number: PCT/US2006/035674
(87) International publication number: WO 2007/035361

(56) References cited:
- US-A- 5 575 813
- US-A- 5 935 155
- US-A1- 2001 037 061
- US-A1- 2002 193 845
- US-A1- 2002 198 573
- US-A1- 2002 198 573
- US-A1- 2003 036 790
- US-A1- 2003 093 066
- US-A1- 2003 158 588
- US-A1- 2003 233 134
- US-A1- 2004 186 533
- US-A1- 2005 196 421
- US-B1- 6 458 157

## Description

### Field of the Invention

The present invention is generally directed to neural stimulation and more specifically to an improved electrode array for subretinal stimulation.

### Background of the Invention

In 1755 LeRoy passed the discharge of a Leyden jar through the orbit of a man who was blind from cataract and the patient saw "flames passing rapidly downwards." Ever since, there has been a fascination with electrically elicited visual perception. The general concept of electrical stimulation of retinal cells to produce these flashes of light or phosphenes has been known for quite some time. Based on these general principles, some early attempts at devising prostheses for aiding the visually impaired have included attaching electrodes to the head or eyelids of patients. While some of these early attempts met with some limited success, these early prosthetic devices were large, bulky and could not produce adequate simulated vision to truly aid the visually impaired.

In the early 1930's, Foerster investigated the effect of electrically stimulating the exposed occipital pole of one cerebral hemisphere. He found that, when a point at the extreme occipital pole was stimulated, the patient perceived a small spot of light directly in front and motionless (a phosphene). Subsequently, Brindley and Lewin (1968) thoroughly studied electrical stimulation of the human occipital (visual) cortex. By varying the stimulation parameters, these investigators described in detail the location of the phosphenes produced relative to the specific region of the occipital cortex stimulated. These experiments demonstrated: (1) the consistent shape and position of phosphenes; (2) that increased stimulation pulse duration made phosphenes brighter; and (3) that there was no detectable interaction between neighboring electrodes which were as close as 2.4 mm apart.

As intraocular surgical techniques have advanced, it has become possible to apply stimulation on small groups and even on individual retinal cells to generate focused phosphenes through devices implanted within the eye itsetf. This has sparked renewed interest in developing methods and apparatus to aid the visually impaired. Specifically, great effort has been expended in the area of intraocular retinal prosthetic devices in an effort to restore vision in cases where blindness is caused by photoreceptor degenerative retinal diseases; such as retinitis pigmentosa and age related macular degeneration which affect millions of people worldwide.

Neural tissue can be artificially stimulated and activated by prosthetic devices that pass pulses of electrical current through electrodes on such a device. The passage of current causes changes in electrical potentials across visual neuronal membranes, which can initiate visual neuron action potentials, which are the means of information transfer in the nervous system.

Based on this mechanism, it is possible to input information into the nervous system by coding the sensory information as a sequence of electrical pulses which are relayed to the nervous system via the prosthetic device. In this way, it is possible to provide artificial sensations including vision.

One typical application of neural tissue stimulation is in the rehabilitation of the blind. Some forms of blindness involve selective loss of the light sensitive transducers of the retina. Other retinal neurons remain viable, however, and may be activated in the manner described above by placement of a prosthetic electrode device on the inner (toward the vitreous) retinal surface (epiretinal). This placement must be mechanically stable, minimize the distance between the device electrodes and the visual neurons, control the electronic field distribution and avoid undue compression of the visual neurons.

In 1986, Bullara (US Pat. No. 4,573,481) patented an electrode assembly for surgical implantation on a nerve. The matrix was silicone with embedded iridium electrodes. The assembly fit around a nerve to stimulate it.

Dawson and Radtke stimulated cat's retina by direct electrical stimulation of the retinal ganglion cell layer. These experimenters placed nine and then fourteen electrodes upon the inner retinal layer (i.e., primarily the ganglion cell layer) of two cats. Their experiments suggested that electrical stimulation of the retina with 30 to 100 µA current resulted in visual cortical responses. These experiments were carried out with needle-shaped electrodes that penetrated the surface of the retina (see also US Pat. No. 4,628,933 to Michelson).

The Michelson '933 apparatus includes an array of photosensitive devices on its surface that are connected to a plurality of electrodes positioned on the opposite surface of the device to stimulate the retina. These electrodes are disposed to form an array similar to a "bed of nails" having conductors which impinge directly on the retina to stimulate the retinal cells. US Patent 4,837,049 to Byers describes spike electrodes for neural stimulation. Each spike electrode pierces neural tissue for better electrical contact. US Patent 5,215,088 to Norman describes an array of spike electrodes for cortical stimulation. Each spike pierces cortical tissue for better electrical contact.

The art of implanting an intraocular prosthetic device to electrically stimulate the retina was advanced with the introduction of retinal tacks in retinal surgery. De Juan, et al. at Duke University Eye Center inserted retinal tacks into retinas in an effort to reattach retinas that had detached from the underlying choroid, which is the source of blood supply for the outer retina and thus the photoreceptors. See, e.g., E. de Juan, et al., 99 Am. J. Ophthalmol. 272 (1985). These retinal tacks have proved to be biocompatible and remain embedded in the retina, and choroid/sclera, effectively pinning the retina against the choroid and the posterior aspects of the globe. Retinal tacks are one way to attach a retinal electrode array to the retina. US Patent 5,109,844 to de Juan describes a flat electrode array placed against the retina for visual stimulation. US Patent 5,935,155 to Humayun describes a retinal prosthesis for use with the flat retinal array described in de Juan.
US 2002/0198573 discloses a retina implant according to the first part of claim 1 and US 5575813 discloses a neural contact structure

### Summary of the Invention

Applicant has proposed a combination of the subretinal and epiretinal methods by placing the electronics external to the eye, entering the eye through the pars plana and then piercing the retina (retinotomy) from inside the eye to stimulate subretinally.

The present invention is an improved electrode array for subretinal stimulation. A hard polymer such as polyimide is biocompatible and strong for supporting an electrode array and supporting traces in a thin flex circuit array. The applicants describe the disadvantage of material such as polyimide is that when made thin, it can cut delicate retina tissue. The application further describes methods of protecting the retina. In the present invention applicant takes advantage of the sharp nature of thin polyimide making a point on the end of the electrode array. This allows the flexible circuit electrode array to be both electrode array and surgical tool to cut the retinal and slide the array under the retina in a single action.

The novel features of the invention are set forth in the appended claims. The invention will be best understood from the following description when read in conjunction with the accompanying drawings.

### Brief Description of the Drawings

**FIG. 1** is a perspective view of the implanted portion of the preferred retinal prosthesis.
**FIG. 2** depicts the flexible circuit array before it is folded and attached to the implanted portion.
**FIG. 3** depicts the flexible circuit array folded.
**FIG. 4** is a fundus photo and flourescein angiogram after 3 month of the preferred trans-retinal implant in a rabbit.
**FIG. 5** illustrates a cross-sectional view of an eye showing the placement of the retinal implant and associated electronics.
**FIG. 6** illustrates a cross-sectional view of a retina showing the tissue layers and placement of the retinal implant in the retina for electrical stimulation of the retina.
**FIG. 7** illustrates a cross-sectional view of an eye showing placement of the retinal implant for drug delivery.
**FIG. 8** illustrates a cross-sectional view of an eye showing the placement of the subretinal implant.
**FIG. 9** illustrates a cross-sectional view of an eye showing the placement of the subretinal implant.
**FIG. 10** illustrates a cross-sectional view of a retina showing the tissue layers and placement of the retinal implant in the retina for drug delivery

### Detailed Description of the Preferred Embodiments

The following description is of the best mode presently contemplated for carrying out the invention. This description is not to be taken in a limiting sense, but is made merely for the purpose of describing the general principles of the invention. The scope of the invention should be determined with reference to the claims.

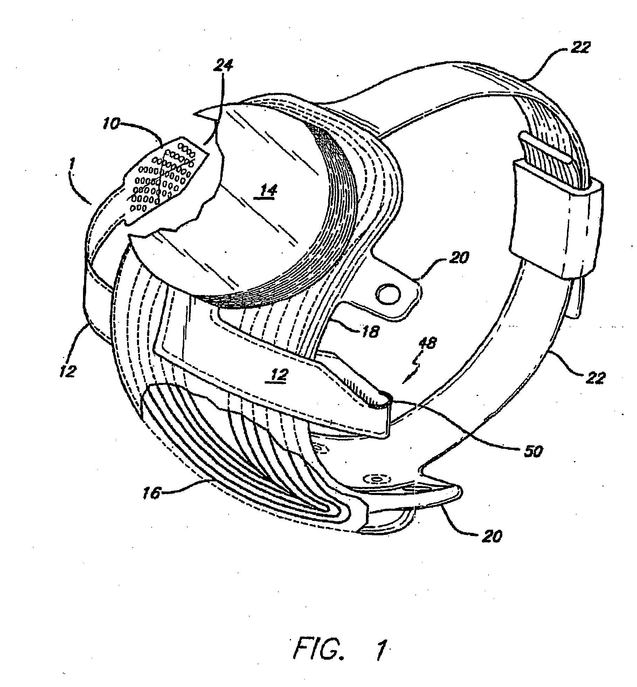

Figure 1 shows a perspective view of the implanted portion of the preferred retinal prosthesis. A flexible circuit 1 includes a flexible circuit electrode array 10. The flexible circuit electrode array 10 is electrically coupled by a flexible circuit cable 12, which pierces first the sclera, then the retina and is electrically coupled to an electronics package 14, external to the sclera. The flexible circuit 1 further forms a point 24 at its end, beyond the flexible circuit electrode array 10. While the point 24 aids in passing the flexible circuit 1 through the sclerotomy, it is particularly useful for passing the flexible circuit 1 through the retina. While the sclera is relatively tough and requires a sclerotomy, the retina is very soft. The point 24 can cut the retina (retinotomy) as in is inserted. This minimizes the size of the retinotomy, reduces the chances of hemorrhage or retina detachment, and simplifies the implant surgery.

The electronics package 14 is electrically coupled to a secondary inductive coil 16. Preferably the secondary inductive coil 16 is made from wound wire. Alternatively, the secondary inductive coil 16 may be made from a flexible circuit polymer sandwich with wire traces deposited between layers of flexible circuit polymer. The electronics package 14 and secondary inductive coil 16 are held together by a molded body 18. The molded body 18 may also include suture tabs 20. The molded body 18 narrows to form a strap 22 which surrounds the sclera and holds the molded body 18, secondary inductive coil 16, and electronics package 14 in place. The molded body 18, suture tabs 20 and strap 22 are preferably an integrated unit made of silicone elastomer. Silicone elastomer can be formed in a pre-curved shape to match the curvature of a typical sclera. However, silicone remains flexible enough to accommodate implantation and to adapt to variations in the curvature of an individual sclera. The secondary inductive coil 16 and molded body 18 are preferably oval shaped. A strap 22 can better support an oval shaped coil.

The flexible circuit 1 is a made by the following process. First, a layer of polymer (such as polyimide, fluoro-polymers, silicone or other polymers) is applied to a support substrate (not part of the array) such as glass. Layers may be applied by spinning, meniscus coating, casting, sputtering or other physical or chemical vapor deposition, or similar process. Subsequently, a metal layer is applied to the polymer. The metal is patterned by photolithographic process. Preferably, a photo-resist is applied and patterned by photolithography followed by a wet etch of the unprotected metal. Alternatively, the metal can be patterned by lift-off technique, laser ablation or direct write techniques.

It is advantageous to make this metal thicker at the electrode and bond pad to improve electrical continuity. This can be accomplished through any of the above methods or electroplating. Then, the top layer of polymer is applied over the metal. Openings in the top layer for electrical contact to the electronics package 14 and the electrodes may be accomplished by laser ablation or reactive ion etching (RIE) or photolithograph and wet etch. Making the electrode openings in the top layer smaller than the electrodes promotes adhesion by avoiding delaminating around the electrode edges.

The implanted portion of the retinal prosthesis includes the additional feature of a gentle twist or fold 48 in the flexible circuit cable 12, where the flexible circuit cable 12 passes through the sclera (sclerotomy). The twist may be a simple sharp twist, or fold 48; or it may be a longer twist, forming a tube. While the tube is rounder, it reduces the flexibility of the flexible circuit. A simple fold 48 reduces the width of the flexible circuit with only minimal impact on flexibility.

Further, silicone or other pliable substance may be used to fill the center of the tube or fold 48 formed by the twisted flexible circuit cable 12. Further it is advantageous to provide a sleeve or coating 50 that promotes healing of the sclerotomy. Polymers, such as polyimide, which may be used to form the flexible circuit cable 12 and flexible circuit electrode array 10, are generally very smooth and do not promote a good bond between the flexible circuit cable 12 and scleral tissue. A sleeve or coating of polyester, collagen, silicone, Gore-tex or similar material would bond with scleral tissue and promote healing. In particular, a porous material will allow scleral tissue to grow into the pores promoting a good bond.

It is also advantageous to create a reverse curve or service loop in the flexible circuit cable 12 of the flexible circuit electrode array to gently lift the flexible circuit cable 12 off the retina and curve it away from the retina, before it pierces the sclera at a sclerotomy. This service loop reduces the likelihood of any stress exerted extraocularly from being transmitted to the electrode region and retina. It also provides for accommodation of a range of eye sizes.

With existing technology, it is necessary to place the implanted control electronics outside of the sclera, while a retinal flexible circuit electrode array must be inside the sclera in order to contact the retina. The sclera is cut through at the pars plana, forming a sclerotomy, and the flexible circuit passed through the sclerotomy. A flexible circuit is thin but wide. The more electrode wires, the wider the flexible circuit must be. It may be difficult to seal a sclerotomy over a flexible circuit wide enough to support enough wires for a high resolution array. A narrow sclerotomy is preferable.

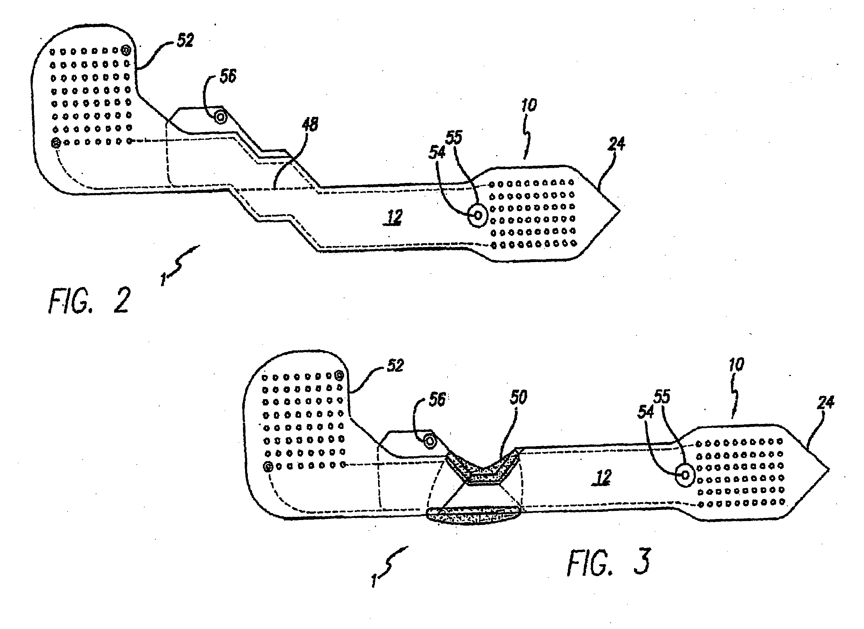

Figure 2 shows the flexible circuit electrode array prior to folding and attaching the array to the electronics package 14. At one end of the flexible circuit cable 12 is an interconnection pad 52 for connection to the electronics package 14. At the other end of the flexible circuit cable 12 is the flexible circuit electrode array 10. Further, a point 24 is provided at the end of the flexible circuit electrode array 10. The flexible circuit cable 12 is formed in a dog leg pattern so than when it is folded at fold 48 it effectively forms a straight flexible circuit cable 12 with a narrower portion at the fold 48 for passing through the sclerotomy.

Figure 3 shows the flexible circuit electrode array after the flexible circuit cable 12 is folded at the fold 48 to form a narrowed section. The flexible circuit cable 12 may include a twist or tube shape as well. With a retinal prosthesis as shown in figure 1, the bond pad 52 for connection to the electronics package 14 and the flexible circuit electrode array 10 are on opposite side of the flexible circuit. This requires patterning, in some manner, both the base polymer layer and the top polymer layer. By folding the flexible circuit cable 12 of the flexible circuit electrode array 10, the openings for the bond pad 52 and the electrodes are on the top polymer layer and only the top polymer layer needs to be patterned. Further, a point 24 is provided at the end of the flexible circuit electrode array 10. The point 24 shown in Figs. 2 and 3 is formed throughout the hole thickness of the array. The array may contain at least one bottom layer containing at least one polymer, copolymer, blockcopolymer or mixtures thereof and at least one top layer containing at least one polymer, copolymer, blockcopolymer or mixtures thereof. The polymer may be polyimide, silicone, PEEK polymer, a repeat unit that comprises of oxy-1, 4-phenylenoeoxy-1, 4-phenylene-carbonyl-1, 4-phenylene, parylene or mixtures thereof.

Also, since the narrowed portion of the flexible circuit cable 12 pierces the sclera, shoulders formed by opposite ends of the narrowed portion help prevent the flexible circuit cable 12 from moving through the sclera. It may be further advantageous to add ribs or bumps of silicone or similar material to the shoulders to further prevent the flexible circuit cable 12 from moving through the sclera.

Further it is advantageous to provide a suture tab 56 in the flexible circuit body near the electronics package to prevent any movement in the electronics package from being transmitted to the flexible circuit electrode array 10. Alternatively, a segment of the flexible circuit cable 12 can be reinforced to permit it to be secured directly with a suture. The retina tack (not shown) is placed through an attachment point 54 to hold the flexible circuit electrode array 10 to the retina. A stress relief 55 can be made of a softer polymer than the flexible circuit 1.

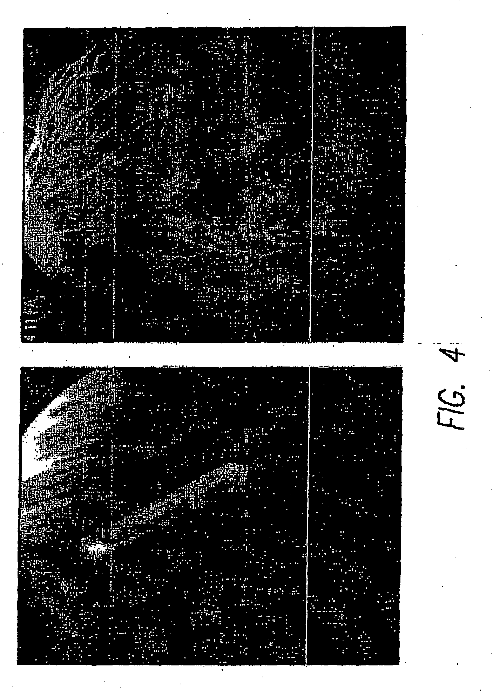

Figure 4 shows a fundus photo and flourescein angiogram after 3 month of the preferred flexible circuit 1 implant in a rabbit. It is shown that the point 24 of the flexible circuit 1 cuts the retina as it is inserted. 15 rabbits were chronically implanted for over three months each with polyimide flexible circuit 1 which were from 0.3 mm to 0.7 mm, preferably about 0.5 mm wide and from 3.5 mm to 4.5 mm, preferably about 4 mm long using the preferred trans-retinal surgical approach which included laser treatment around the retinotomy site. The flexible circuit 1 were inserted under the retina and left with a portion of the flexible circuit cable 12 sticking out into the vitreous. In an actual device, according the present invention, the flex circuit cable 12 of figure 4 would be attached to an electronics package 14 as described above.

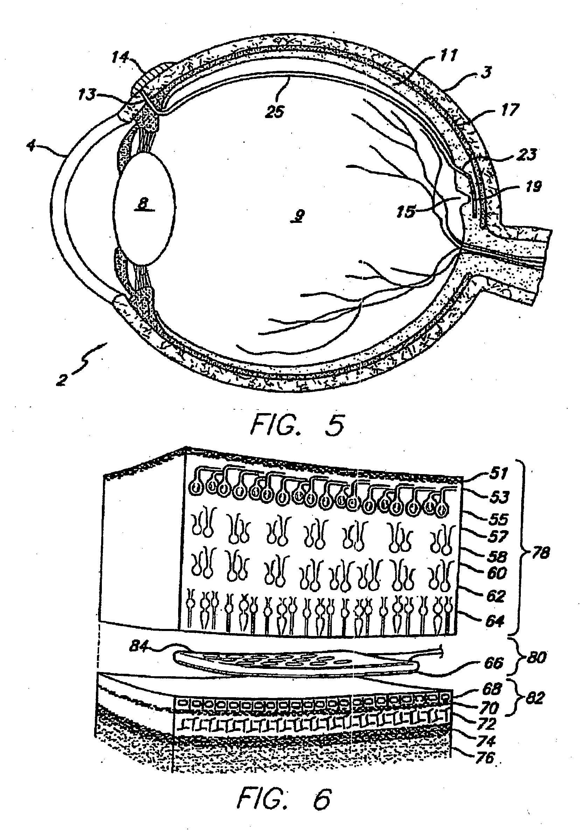

Figure 5 provides a cross-sectional view of a preferred embodiment of the eye 2 with a retinal implant 19 placed subretinally. The current invention involves the use of an electronic device, a retinal implant 19 that is capable of mimicking the signals that would be produced by a normal inner retinal photoreceptor layer. When the device is implanted subretinally between the inner and outer retinal layers, it will stimulate the inner layer to provide significantly useful formed vision to a patient who's eye no longer reacts to normal incident light on the retina 20. Patient's having a variety of retinal diseases that cause vision loss or blindness by destruction of the vascular layers of the eye, including the choroid, choriocapillaris, and the outer retinal layers, including Bruch's membrane and retinal pigment epithelium. Loss of these layers is followed by degeneration of the outer portion of the inner retina, beginning with the photoreceptor layer. The inner retina, composed of the outer nuclear, outer plexiform, inner nuclear, inner plexiform, ganglion cell and nerve fiber layers, may remain functional. Functioning of the inner retina allows electrical stimulation of this structure to produce sensations of light or even vision.

The biocompatible retinal implant 19 is attached by an electrically conductive cable or lead wire 25 that is also biocompatible, to a control electronics 14 package that contains suitable electronics to generate an electrical signal that is transmitted along a lead wire 25 to the retinal implant, which stimulates the retina 11. The lead wire 25 passes transretinally through retinal incision 13 and enters the vitreous cavity 9. The lead wire 25 then passes transsclera at sclera incision 13 that passes through the sclera at a location near the front of the eye where there is no retina 11.

The eye 2 has a cornea 4, lens 8, and vitreous cavity 9 through which light normally passes, prior to striking the retina 11 and causing vision. The eye 2 has an outer layer, called the sclera 6, and a nutrient rich layer, called the choroid 18, that is located between the retina 11 and the sclera 6.

In a preferred embodiment, the retinal implant 20 is located subretinally near the fovea 15 to provide good electrical contact between the retinal implant 19 and the retina 11. The lead wire 25, which is attached to the retinal implant 19, proceeds transretinally through retina 11 via retinal incision 23. Passing the lead wire into the vitreous cavity 9 via the retinal incision 23 avoids disrupting the delicate choroid 17, and thereby avoids interfering with the supply of nutrients to the retina 11. The lead wire 25 passes through the vitreous cavity to a point near the front of the eye 2 where it traverses transsclera via an incision13 through the sclera 6 at a point where the retina 11 and choroid 17 are not present, thereby further avoiding disruption to the blood supply, oxygen, and nutrients that are needed to sustain the retina 11. While the choroid 17 does extend to this region of the eye near the lens 8, called the pars plana, choroid 17 bleeding will not damage the retina 11, and is far less likely to spread to the central retina 11, called the macula, which is the area of most sensitive vision, while choroid 17 bleeding under the retina 11 can track along the retina 11 and end up damaging the macular region near the fovea 19 of the retina 11.

The control electronics 14 are located outside the eye 2 and are attached to lead wire 25. The control electronics 14 are preferably attached to the sclera 6 by sutures. In alternative embodiments, the control electronics 14 are located distant from the eye 2.

A perspective cross-sectional view of the retina and outer wall of the eye is presented in Figure 6. Moving from the inside of the eye outward, the structure of the eye is encountered as follows: internal limiting membrane 51, axons 53, ganglion and amacrine cell layer 55, inner plexiform 57, inner nuclear layer 58, outer plexiform layer 60, bipolar cell layer 62, photoreceptor cell layer 64, retinal pigment epithelium 68, Bruck's membrane 70, choriocapillaris 72, choroid 74, and the outer coat or sclera 76.

The inner retina 78 is generally the structures from the internal limiting membrane 50 to the photoreceptor cell layer 64. The outer retinal layer is the retinal pigment epithelium 68 and Bruck's membrane 70.

A subretinal implant position 80 is located between the photoreceptor cell layer 64 and the retinal pigment epithelium 68. In a preferred embodiment, the retinal implant 66 is surgically implanted in the subretinal implant position 80.

In a preferred embodiment, the retinal implant 66 is biocompatible and contains a number of arrayed electrodes 84, which are electrically stimulated by an outside source to stimulate the inner retinal layer 78, thereby to provide significantly useful formed vision. It is preferred that the electrodes 84 are located on the surface of the retinal implant 66 that faces the front of the eye, to stimulate the inner retinal layer 78.

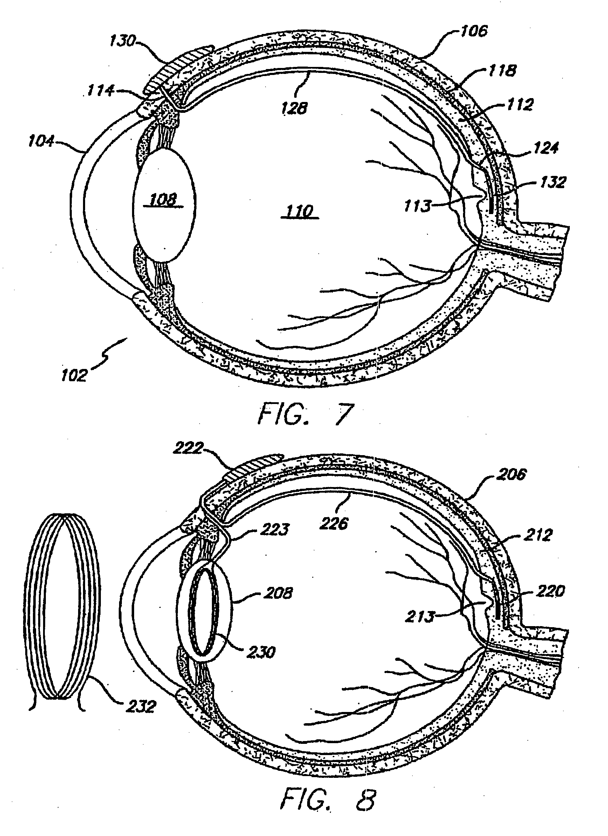

A cross-sectional view of the eye 102 and retinal implant 132 is presented in Figure 7. In this embodiment of the invention, drugs are delivered by transfer from drug reservoir 130 to retinal implant 132, where the drugs are released subretinally for treatment of the tissue of the eye 2 and especially the retinal tissue. This device is particularly advantageous for treatment of chronic issues. A further advantage is that the quantity of drugs required and released to the eye is minimized by releasing the drugs in near proximity to the area of the eye 102 that requires treatment.

In a preferred embodiment, the drugs are transferred from drug reservoir 130 via delivery conduit 128, which is preferably a tube, to retinal implant 132. While the drugs may be pumped or delivered by other known means, it is preferable that they be delivered electrophoretically.

The structure of the eye 2, as shown in Figure 7, presents a cornea 104 at the front of the eye with a lens 108 behind. The sclera 106 is on the outside of the eye and the choroid 118 is inside the eye 2 between the retina 112 and sclera 106.

The retinal implant 132 is implanted subretinally, preferably near the back of the eye. It is shown near the fovea 113, in Figure 7, but may be located at other subretinal locations, as desired. The drug delivery conduit 128 connects the retinal implant 132 with the drug reservoir 130. The conduit 128 passes transretinally through retinal incision 124 and enters the vitreous cavity 110. The conduit 128 then passes transsclera at sclera incision 114 that passes through the sclera at a location near the front of the eye where there is no retina 112, thereby avoiding damage to the nutrient rich choroid 118 and avoiding disruption of the blood supply to the retina 112.

An alternative embodiment of a retinal implant to enable vision restoration is presented in Figure 8, wherein a cross-section of the eye is presented showing the lens 208, retina 212, sclera 206, and fovea 213. U.S. Patent No. 5,935,155, issued to Humayun, et al., the '155 patent, describes a similar visual prosthesis and method of use. In this embodiment, the retinal implant 220 is implanted subretinally. A primary coil 232 is located preferably either in an eyeglass lens frame or in a soft contact lens. This coil 232 is used to inductively couple the radio frequency encoded image signal to the secondary coil 230 that, in this embodiment, is implanted behind the iris of the eye. The control electronics 222 is placed in a hermetically sealed package and is coupled to a secondary coil 230 by a coil lead 223 that pierces the sclera 206 at a point near the lens 208 where there is no retina 212. The control electronics 222 is attached to the outside of the sclera 206. A lead wire 226 coupling the control electronics 222 to the retinal implant 220 passes transsclera at a point where there is no retina, preferably near the lens 208. The lead wire 226 passes inside the eye, preferably along the interior wall of the eye, and pierces the retina to pass transretinal to couple the control electronics 222 to the retinal implant 220. This invention is an improvement over that disclosed by the '155 patent because the retinal implant is subretinal rather than epiretinal, thereby facilitating stimulation of the retinal tissue.

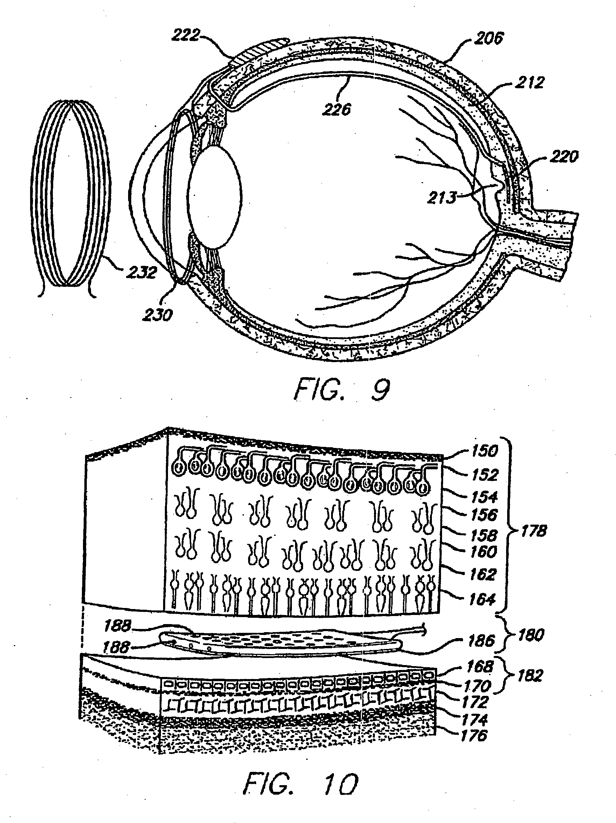

A further alternative embodiment of a retinal implant to enable vision restoration is presented in Figure 9. The `155 patent discloses a similar invention, wherein the retinal implant 220 is placed subretinally. In this embodiment, the secondary 230 is attached to the sclera 206 instead of being implanted within the eye. As with the control electronics 222, the attachment of the secondary coil 230 to the sclera 206 may be by suturing or other appropriate means, as discussed in the `155 patent. In this way, only the lead wire 226 which attaches the control electronics 222 to the retinal implant 220 mounted subretinally below retina 212 is required to pierce the sclera 206. The extra-ocular attachment of the control electronics 222 allows increased access to this circuitry that eases the replacement or updating of these components.

Figure 10 presents a perspective cross-sectional view of the retina and outer wall of the eye. The tissue layers from the inside of the eye outward are the internal limiting membrane 150, axons 152, ganglion and amacrine cell layer 154, inner plexiform 156, inner nuclear layer 158, outer plexiform layer 160, bipolar cell layer 162, photoreceptor cell layer 164, retinal pigment epithelium 168, Bruck's membrane 170, choriocapillaris 172, choroid 174, and sclera 176.

The inner retinal layer 178 is comprised of tissue from the internal limiting membrane 150 to the photoreceptor cell layer 164. The outer retinal layer 182 consists of the retinal pigment epithelium 168 and Bruck's membrane 170.

Between the inner retinal layer 178 and outer retinal layer 182, is the subretinal implant position 180 in which retinal implant 186 is surgically located.

The retinal implant contains a number of orifices 188 through with the drug is released into the surrounding retinal tissue. The orifices 188 are preferably uniformly presented on both the inner and outer surfaces as well as on the edges of the retinal implant 186. However, the orifices 188 may be preferentially oriented in the retinal implant 186 to selectively release the drug on or near a desired tissue or location.

Accordingly, what nas been shown is an improved method making a neural electrode array and improved method of stimulating neural tissue. While the invention has been described by means of specific embodiments and applications thereof, it is understood that numerous modifications and variations could be made thereto by those skilled in the art without departing from the scope of the invention. It is therefore to be understood that within the scope of the claims, the invention may be practiced otherwise than as specifically described herein.

## Claims

1. A flexible circuit electrode array adapted for subretinal stimulation, comprising:
a polymer base layer;
metal traces deposited on said polymer base layer, including electrodes suitable to stimulate neural tissue; and
a polymer top layer deposited on said polymer base layer and said metal traces;
**characterized in that** said
polymer base layer, said metal traces and said polymer top layer are curved to approximate the curvature of an eye,
said polymer top layer is on a concave side of said curved polymer base layer and said metal traces and defines openings for said electrodes (84) on said concave side of said flexible circuit electrode array (10); and **in that**
said polymer base layer and said polymer top layer are pointed to aid in forming retinotomy.

2. The flexible circuit electrode array (10) according to claim 1, wherein said polymer top layer defines openings for bond pads on the same side of said flexible circuit electrode array (10) as said openings for said electrodes (84).

3. The flexible circuit electrode array (10) according to claim 1, further comprising at least one bumper bonded to a peripheral edge of said flexible circuit electrode array (10).

4. The flexible circuit electrode array (10) according to claim 1, further comprising a narrowed portion in a flexible circuit cable portion (12) of said flexible circuit electrode array (10).

5. The flexible circuit electrode array (10) according to claim 1, wherein said polymer top layer is a more compliant polymer than said curved polymer base layer.

6. The flexible circuit electrode array (10) according to claim 1, further comprising a stress relief membrane suitable for attachment of said flexible circuit electrode array (10), wherein said stress relief membrane is a more compliant material than said curved polymer base layer.

7. The flexible circuit electrode array (10) according to claim 1, further comprising a narrowed portion in a flexible circuit cable portion (12) of said flexible circuit electrode array (10).

8. The flexible circuit electrode array (10) according to claim 7, wherein said narrowed portion is suitable to pierce a sclera (6).

9. The flexible circuit electrode array (10) according to claim 1, further comprising diagonal fold (48) in a flexible circuit cable portion (12) of said flexible circuit electrode array (10).

10. The flexible circuit electrode array (10) according to claim 9, where said diagonal fold (48) is across a dogleg in said flexible circuit electrode array (10).

11. The flexible circuit electrode array (10) according to claim 7, further comprising bond pads coupled to said metal traces on an end of said flexible circuit electrode array (10) opposite to said electrodes (84) and openings in said polymer top layer for said electrodes (84) and said bond pads.

12. The flexible circuit electrode array (10) according to claim 7, wherein said fold (48) forms a narrowed portion.

13. The flexible circuit electrode array (10) according to claim 1, further comprising a widened portion of a flexible circuit cable portion (12) of said flexible circuit electrode array (10) suitable to resist movement of said flexible circuit electrode array (10).

14. An implantable device to affect an eye comprising a flexible circuit electrode array (10) according to claim 1.

## Patentansprüche

1. Flexible Schaltkreis-Elektrodenanordnung, die zur subretinalen Stimulierung geeignet ist und die Folgendes umfasst:
eine Polymerbasisschicht;
Metallbahnen, die auf der Polymerbasisschicht aufgebracht sind und Elektroden umfassen, die für die Stimulierung von Nervengewebe geeignet sind; und
eine Polymeroberschicht, die auf der Polymerbasisschicht und den Metallbahnen aufgebracht ist;
**dadurch gekennzeichnet, dass**
die Polymerbasisschicht, die Metallbahnen und die Polymeroberschicht ungefähr mit der Krümmung eines Auges gekrümmt sind,
wobei die Polymeroberschicht auf einer konkaven Seite der gekrümmten Polymerbasisschicht und der Metallbahnen ist und Öffnungen für die Elektroden (84) auf der konkaven Seite der flexiblen Schaltkreis-Elektrodenanordnung (10) definiert; und worin
die Polymerbasisschicht und die Polymeroberschicht spitz zulaufend sind, um die Bildung einer Retinotomie zu unterstützen.

2. Flexible Schaltkreis-Elektrodenanordnung (10) nach Anspruch 1, worin die Polymeroberschicht Öffnungen für Haftverbindungskissen auf derselben Seite der flexiblen Schaltkreis-Elektrodenanordnung (10) wie die Öffnungen für die Elektroden (84) definiert.

3. Flexible Schaltkreis-Elektrodenanordnung (10) nach Anspruch 1, wobei diese ferner zumindest eine Distanzleiste umfasst, die mit einer Umfangskante der flexiblen Schaltkreis-Elektrodenanordnung (10) haftschlüssig verbunden ist.

4. Flexible Schaltkreis-Elektrodenanordnung (10) nach Anspruch 1, wobei diese ferner einen geschmälerten Abschnitt in einem flexiblen Schaltkreisabschnitt (12) der flexiblen Schaltkreis-Elektrodenanordnung (10) umfasst.

5. Flexible Schaltkreis-Elektrodenanordnung (10) nach Anspruch 1, worin die Polymeroberschicht ein nachiebigeres Polymer ist als die gekrümmte Polymerbasisschicht.

6. Flexible Schaltkreis-Elektrodenanordnung (10) nach Anspruch 1, wobei diese ferner eine Spannungsverringerungsmembran umfasst, die zur Befestigung der flexiblen Schaltkreis-Elektrodenanordnung (10) geeignet ist, worin die Spannungsverringerungsmembran ein nachgiebigeres Material ist als die gekrümmte Polymerbasisschicht.

7. Flexible Schaltkreis-Elektrodenanordnung (10) nach Anspruch 1, wobei diese ferner einen geschmälerten Abschnitt in einem flexiblen Schaltkreiskabelabschnitt (12) der flexiblen Schaltkreis-Elektrodenanordnung (10) umfasst.

8. Flexible Schaltkreis-Elektrodenanordnung (10) nach Anspruch 7, worin der geschmälerte Abschnitt dazu geeignet ist, eine Sklera (6) zu durchbohren.

9. Flexible Schaltkreis-Elektrodenanordnung (10) nach Anspruch 1, wobei diese ferner eine Diagonalfaltung (48) in einem flexiblen Schaltkreiskabelabschnitt (12) der flexiblen Schaltkreis-Elektrodenanordnung (10) umfasst.

10. Flexible Schaltkreis-Elektrodenanordnung (10) nach Anspruch 9, wobei die Diagonalfaltung (48) quer über einer Oberflächenbeschädigung in der flexiblen Schaltkreis-Elektrodenanordnung (10) verläuft.

11. Flexible Schaltkreis-Elektrodenanordnung (10) nach Anspruch 7, wobei diese ferner Haftverbindungskissen umfasst, die an die Metallbahnen auf einem Ende der flexiblen Schaltkreis-Elektrodenanordnung (10) gegenüber den Elektroden (48) und den Öffnungen in der Polymeroberschicht für die Elektroden (84) und die Haftverbindungskissen gekoppelt sind.

12. Flexible Schaltkreis-Elektrodenanordnung (10) nach Anspruch 7, worin die Faltung (48) einen geschmälerten Abschnitt bildet.

13. Flexible Schaltkreis-Elektrodenanordnung (10) nach Anspruch 1, wobei diese ferner einen erweiterten Abschnitt eines flexiblen Schaltkreiskabelabschnitts (12) der flexiblen Schaltkreis-Elektrodenanordnung (10) umfasst, der dazu geeignet ist, Bewegungen der flexiblen Schaltkreis-Elektrodenanordnung (10) standzuhalten.

14. Implantierbare Vorrichtung, um ein Auge zu beeinflussen, das eine flexible Schaltkreis-Elektrodenanordnung (10) nach Anspruch 1 umfasst.

## Revendications

1. Réseau d'électrodes à circuit souple conçu pour une stimulation sous-rétinienne, comprenant :
une couche de base polymérique ;
des pistes métalliques déposées sur ladite couche de base polymérique, comprenant des électrodes appropriées pour stimuler un tissu neuronal ; et
une couche supérieure polymérique déposée sur ladite couche de base polymérique et lesdites pistes métalliques ;
**caractérisé en ce que**
ladite couche de base polymérique, lesdites pistes métalliques et ladite couche supérieure polymérique sont incurvées pour présenter approximativement la courbure d'un oeil,
ladite couche supérieure polymérique est sur un côté concave de ladite couche de base polymérique incurvée et desdites pistes métalliques et définit des ouvertures pour lesdites électrodes (84) sur ledit côté concave dudit réseau d'électrodes à circuit souple (10) ; et **en ce que**
ladite couche de base polymérique et ladite couche supérieure polymérique sont destinées à aider à la formation d'une rétinotomie.

2. Réseau d'électrodes à circuit souple (10) selon la revendication 1, dans lequel ladite couche supérieure polymérique définit des ouvertures pour des pastilles de liaison du même côté dudit réseau d'électrodes à circuit souple (10) que lesdites ouvertures pour lesdites électrodes (84).

3. Réseau d'électrodes à circuit souple (10) selon la revendication 1, comprenant en outre au moins un élément d'amortissement lié à un bord périphérique dudit réseau d'électrodes à circuit souple (10).

4. Réseau d'électrodes à circuit souple (10) selon la revendication 1, comprenant en outre une partie rétrécie dans une partie de câble de circuit souple (12) dudit réseau d'électrodes à circuit souple (10).

5. Réseau d'électrodes à circuit souple (10) selon la revendication 1, dans lequel ladite couche supérieure polymérique est un polymère plus souple que ladite couche de base polymérique incurvée.

6. Réseau d'électrodes à circuit souple (10) selon la revendication 1, comprenant en outre une membrane de relâchement des contraintes appropriée pour la fixation dudit réseau d'électrodes à circuit souple (10), dans lequel ladite membrane de relâchement des contraintes est en un matériau plus souple que ladite couche de base polymérique incurvée.

7. Réseau d'électrodes à circuit souple (10) selon la revendication 1, comprenant en outre une partie rétrécie dans une partie de câble de circuit souple (12) dudit réseau d'électrodes à circuit souple (10).

8. Réseau d'électrodes à circuit souple (10) selon la revendication 7, dans lequel ladite partie rétrécie est appropriée pour percer une sclérotique (6).

9. Réseau d'électrodes à circuit souple (10) selon la revendication 1, comprenant en outre un pli diagonal (48) dans une partie de câble de circuit souple (12) dudit réseau d'électrodes à circuit souple (10).

10. Réseau d'électrodes à circuit souple (10) selon la revendication 9, dans lequel ledit pli diagonal (48) est en travers d'un coude dans ledit réseau d'électrodes à circuit souple (10).

11. Réseau d'électrodes à circuit souple (10) selon la revendication 7, comprenant en outre des pastilles de liaison couplées aux dites pistes métalliques sur une extrémité dudit réseau d'électrodes à circuit souple (10) opposée aux dites électrodes (84) et des ouvertures dans ladite couche supérieure polymérique pour lesdites électrodes (84) et lesdites pastilles de liaison.

12. Réseau d'électrodes à circuit souple (10) selon la revendication 7, dans lequel ledit pli (48) forme une partie rétrécie.

13. Réseau d'électrodes à circuit souple (10) selon la revendication 1, comprenant en outre une partie élargie d'une partie de câble de circuit souple (12) dudit réseau d'électrodes à circuit souple (10) appropriée pour résister à un déplacement dudit réseau d'électrodes à circuit souple (10).

14. Dispositif implantable pour affecter un oeil comprenant un réseau d'électrodes à circuit souple (10) selon la revendication 1.
